(19) Europäisches Patentamt
European Patent Office
Office européen des brevets

(11) EP 3 409 294 A1

(12) EUROPEAN PATENT APPLICATION

(43) Date of publication:
**05.12.2018 Bulletin 2018/49**

(21) Application number: **17461543.5**

(22) Date of filing: **01.06.2017**

(51) Int Cl.:
***A61K 47/38*** *(2006.01)*
***A61K 9/20*** *(2006.01)*
***A61K 9/28*** *(2006.01)*
***A61K 31/4709*** *(2006.01)*
***A61K 9/14*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **PRZEDSIEBIORSTWO FARMACEUTYCZNE LEK-AM SP Z O. O.
05-170 Zakroczym (PL)**

(72) Inventors:
- **LECHNIO, Joanna
00-195 Warszawa (PL)**
- **WI NIEWSKA, Anna
01-873 Warszawa (PL)**
- **NICHTHAUSER, Joanna
01-793 Warszawa (PL)**
- **URBA SKA, Agnieszka
05-110 Jab onna (PL)**
- **OSINSKI, Andrzej
00-875 Warszawa (PL)**
- **HEJDUK, Arkadiusz
05-462 Wi zowna (PL)**
- **MILANOWSKI, Bart omiej
63-200 Jarocin (PL)**
- **JAKUBOWSKA, Emilia
61-531 Pozna (PL)**
- **LULEK, Janina
62-010 Pobiedziska (PL)**

(74) Representative: **BRANDPAT Patent and Trademark Attorneys
Chlebicka Czyz Galazkiewicz Ziolkowski
Professional Partnership
Hoza 29/31 Apt.31
00-521 Warsaw (PL)**

# (54) TABLETS CONTAINING CILOSTAZOL OF SPECIFIC PARTICLE SIZE DISTRIBUTION

(57) Pharmaceutical tablet composition comprising a pharmaceutically acceptable ingredient - cilostazol in the form of particles having the following particle size distribution:

$D_{0.5}$ 10-30 μm
$D_{0.9}$ 40-70 μm and bioavailability of the commercial product reference drug Pletal

Fig. 2

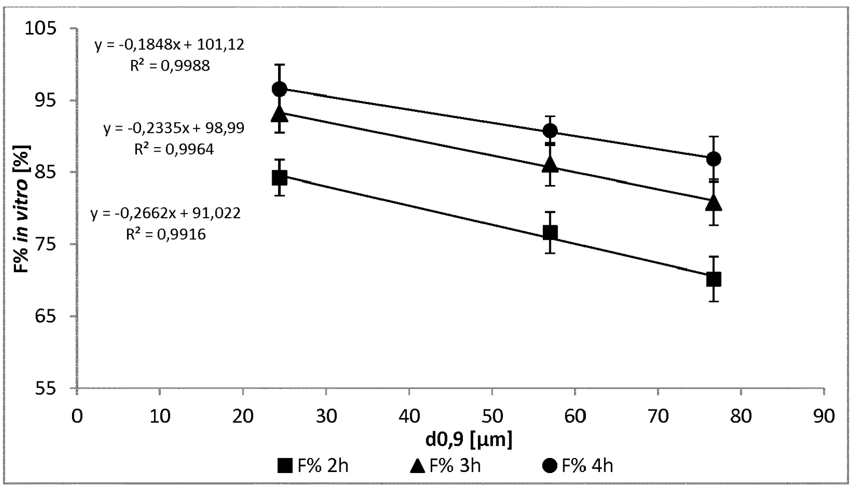


EP 3 409 294 A1

## Description

The invention refers to cilostazol tablets having cilostazol particles of specific particle size distribution as well as medicinal product comprising cilostazol as active ingredient, with bioequivalence responding to the original drug Pletal (Otsuka Pharmaceutical Europe Ltd.).

## Technical Field

The active substance of the medicinal product, ***Cilostazol*** of formula I, (general name of 6-[4-(1-cyclohexyl-1H-tetrazol-5-yl)butoxy]-3,4-dihydrocarbostyryl) is a 2-oxo-quinoline derivative with ATC code B01AC23.

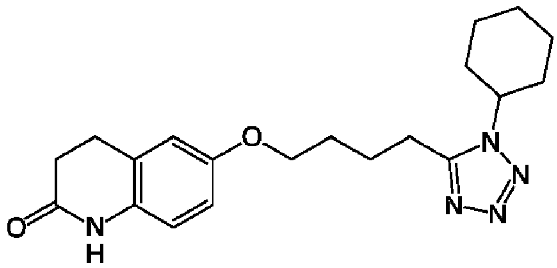

I

Cilostazol selectively inhibits III phosphodiesterase (PDE 3) activity and suppresses cyclic adenosine-3',5'-monophosphate (cAMP) degradation with a resultant increase in cAMP concentration in platelets and blood vessels, leading to inhibition of platelet aggregation and vasodilation.

Cilostazol is a low solubility drug and therefore, to achieve the desired dissolution profile and bioavailability from cilostazol compositions, it requires well-defined dissolution rate improving approaches.

## Background Art

EP 2481398 A1 (European phase of WO2011037281) discloses a slow-release tablet including cilostazol as a pharmacologically active component, with an extended elution time, for suppressing aggregation of blood platelets and promoting vascular relaxation. The slow-release cilostazol tablet comprises a release-controlling polymer, a binder, a filler and a lubricant. The slow-release cilostazol tablet maintains a constant elution rate even with changes in pH in the stomach and intestines.

EP2273981 (European phase of WO2009139504) relates to a sustained release solid pharmaceutical formulation comprising (a) cilostazol as the active medical ingredient, (b) a pre-gelatinized starch in an amount of 10 to 90 % by weight based on the whole weight of the formulation, (c) one or more kinds of enteric ingredients and (d) an organic acid. The kinds of enteric ingredients comprise hydroxypropyl methylcellulose phthalate, hydroxypropyl methylcellulose acetate succinate, carboxymethylethylcellulose, methacrylic acid copolymer L and/or methacrylic acid copolymer S. The formulation is aimed at decreasing administration frequency by providing sustained release, together with reduction of side-effects.

EP1755682 (WO2005113009) discloses a solid pharmaceutical sustained release formulation which comprises (a) an active medical ingredient cilostazol and (b) a pre-gelatinized starch in an amount of 10 to 90 % by weight based on the whole weight of the formulation.

EP1300160 B1 discloses a solid pharmaceutical preparation comprising (1) a pharmacological substance Cilostazol, (2) sodium lauryl sulfate, magnesium stearate and (3) sodium citrate or magnesium oxide. The preparation is declared to be excellent in storage stability and suppressed in discoloration and deterioration.

EP2262487 (WO2009107864) provides an orally disintegrating tablet comprising cilostazol, granulated particles which are prepared by uniformly dispersing an inorganic substance and a disintegrating agent into complex particles comprising two or more kinds of saccharides, a glidant, and an organic excipient, which may further optionally comprise one or more kinds of additives selected from the group consisting of lubricants, sweeteners, flavoring substances, flavors, binders and colorants. The tablet exhibits a good disintegrability in the oral cavity and a pleasant feeling in a mouth when administered and also has a sufficient hardness.

EP1162973 (WO0057881) discloses a cilostazol preparation having a capability of dissolving cilostazol even at the lower portion of the digestive tract, which comprises incorporating a fine powder of cilostazol, preferably having average particle diameter of 10μm or less, into a dispersing and/or solubilizing agent selected from the group consisting of a water-soluble polymer, a surfactant and a mixture thereof.

EP1802304 (WO2006030301) is providing a pharmaceutical composition that includes cilostazol particles

wherein at least 90% of the cilostazol particles are less than about 50 μm. The embodiments of the composition may include one or more of the following features. For example, at least 90% of the cilostazol particles may be less than about 50 μm and at least 25% of the cilostazol particles may be greater than about 15 μm. At least 90% of the cilostazol particles may be less than about 45 μm and at least 50% cilostazol particles may be greater than about 15 μm. The cilostazol particles may be prepared by a process of milling. The cilostazol may be blended with a carrier and milled to form a co-milled mass of the particle size.

However, there is still a need for cilostazol formulations that are bioequivalent when compared to the existing marketed compositions, their process of manufacture is simple, and also have reproducible performance when compared to the existing compositions. None of the above prior art documents teach or suggest the perfect solution to the aforementioned problem.

## Summary of Invention

The invention provides a pharmaceutical tablet composition comprising a pharmaceutically acceptable active pharmaceutical ingredient (API) - cilostazol in the form of particles having the following particle size distribution:

$D_{0.5}$ 10-30 μm

$D_{0.9}$ 40-70 μm, preferably $D_{0.9}$ 50-70 μm, more preferably $D_{0.9}$ 50-60 μm,

and bioavailability of the commercial product reference drug Pletal.

The composition comprises at least one pharmaceutically acceptable excipient selected from the group of fillers, disintegrants, binders, lubricants.

A filler can be selected from: cellulose microcrystalline, powdered cellulose, lactose anhydrous, lactose monohydrate, mannitol, sorbitol, calcium phosphate dibasic anhydrous, calcium phosphate dibasic dihydrate; preferably cellulose microcrystalline.

A disintegrant can be selected from sodium starch glycolate, croscarmellose or its salt, crospovidone, hydroxypropyl cellulose low-substituted, starch, starch pregelatinised; preferably croscarmellose sodium.

A binder can be selected from povidone, hydroxypropylcellulose, hydroxypropyl methylcellulose (hypromellose); preferably hydroxypropyl methylcellulose.

A lubricant can be selected from stearic acid, stearic acid salts such as magnesium stearate, sodium lauryl sulfate, hydrogenated vegetable oil, hydrogenated castor oil; preferably magnesium stearate.

The composition of the invention is obtained by tableting the granulate manufactured in wet- granulation process.

## Technical Problem

The particles of active ingredient cilostazol having the following particle size distribution:

*$D_{0.5}$ 20-40 μm*

$D_{0.9}$ 75-95 μm.

formulate a medicinal product of insufficient bioavailability. Clinical data not published.

At the same time, particles of active ingredient cilostazol with the following particle size distribution:

$D_{0.5}$ 5-15μm

$D_{0.9}$ 20 -40 μm.

when applied into medicinal product, result with the final product having too high bioavailability in comparison to a reference drug. Data not published.

Therefore the aim of the invention is to indicate particle size of cilostazol which is necessary to create immediate release generic drug medicinal product with bioavailability comparable to the reference drug Pletal (Otsuka Pharmaceutical Europe Ltd.)

## Solution to Problem

The invention provides a solution to the problem how to appoint cilostazol particles size in immediate release

formulation (generic medicinal product) with acceptable bioavailability.

A generic medicinal product is a product which has the same quantitative and qualitative composition in active substances and the same pharmaceutical form as the reference medicinal product, and whose bioequivalence with the reference medicinal product has been demonstrated by appropriate bioavailability studies.

According to Guideline on the Investigation of Bioequivalence CPMP/EWP/QWP/1401/98 Rev. 1 /Corr** two medicinal products containing the same active substance are considered bioequivalent if they are pharmaceutically equivalent or pharmaceutical alternatives and their bioavailabilities (rate and extent) after administration in the same molar dose lie within acceptable predefined limits. These limits are set to ensure comparable *in vivo* performance, i.e. similarity in terms of safety and efficacy.

Pharmacokinetic parameters to be investigated during bioequivalence studies are as follows:

AUC - area under the plasma concentration curve

$C_{max}$ - maximum plasma concentration

$t_{max}$ - time until $c_{max}$ is reached

In order to determine bioequivalence after a single dose, the parameters to be analysed are $C_{max}$, $AUC_{(0-1)}$, or when relevant, $AUC_{(0-72h)}$. For these parameters the 90% confidence interval for the ratio of the test and reference products values should be contained within the acceptance interval of 80.00-125.00%.

As a part of the conducted studies, mathematical equations describing the *in vitro - in vivo* correlation (IVIVC) were developed, i.e. the relationship between the results of clinical trials (*in vivo),* and the results of drug release during laboratory stage (*in vitro*). To ensure the accuracy of the model, it should be based on the results of release and bioequivalence studies of at least 3 series of test product and / or reference product.

Estimation of an *in vitro - in vivo* correlation helps to predict the bioavailability of other series of the product without conducting clinical trials (within a specified scope), based solely on the results of their *in vitro* release.

As a result of the conducted studies, the IVIVC equation was developed by using the results of clinical and laboratory trials of the reference drug Pletal s. 5564803 and test products of two bioequivalence studies: s. CSB021014 and CSB010715. The results of release of CSB010716 tablets, which were not targeted for clinical trials, were also available. Based on the *in vitro* results of this series, the bioavailability which would be expected in the case of its *in vivo* studies was estimated.

The ratio of $C_{max}$ of test product and reference product $C_{max}$ T/R [%]) was used as a characterizing clinical parameter of the bioavailability of the various products. The results are shown in Table 1.

Table 1. Products and API (Active Pharmaceutical Ingredient) series used for development of *in vitro - in vivo* correlation

| **Trial No / Product** | **Obtained in accordance with** | **API batches (percentage)** | **API particle size distribution [μm]** | | ***In vivo* results $c_{max}$ T/R [%] (90% confidence interval)** |
|---|---|---|---|---|---|
| | | | $d_{0,5}$ | $d_{0,9}$ | |
| **Trial No 1** Test Product Batch No. CSB021014 vs. Pletal Batch No. 5564803 | Example 4 | 06140376 (100%) | 28.09 | 76.68 | *Below 80* |
| **Trial No 2** Test Product Batch No. CSB010715 vs. Pletal Batch No. 5564803 | Example 4 | 061502440(93,3%:) + 061502467: (6,7%) | 17.86 | 57.00 | *80-125* |
| Test Product Batch No. CSB010716 | Example 4 | 061502467/IF (100%) | 6.57 | 24.39 | |

The test product targeted to trial No. 1 was manufactured with active substance characterized by particle size distribution as follows: $d_{0.5}$ = 28.09 μm; $d_{0.9}$ = 76.682 μm The ratio $C_{max}$ T/R was below 80 %. It means, that bioequivalence was not obtained.

The test product targeted to trial No. 2 was manufactured with active substance characterized by particles size distribution as follows: $d_{0.5}$ = 17.86 μm; $d_{0.9}$ = 57.00 μm. The ratio $C_{max}$ T/R was within the range 80.00-125.00 %. It

means that bioequivalence was proved.

For both mentioned above batches of the tested product, as well as for reference product, dissolution profiles were checked in a flow-through cell apparatus (Apparatus 4). Test conditions were as follows: medium: 0.3% SDS, 12mm chamber diameter, open flow = 8mL/min, the release of Cilostazol was determined by UV spectrophotometry in on-line mode. The same conditions were applied to the testing of batch CSB010716, which was not intended for the clinical trials.

As a result, the correlation equation between *in vivo* values and the amount of Cilostazol released from the individual products within 2, 3 and 4 hours (*F% 2h, F% 3h, F% 4h*) was obtained. Moreover, a mathematical correlation between the amount of cilostazol released *in vitro* and the particle size of the API used for the manufacturing of the 3 generic product batches (CSB021014, CSB010715 and CSB010716) has been found. The established mathematical models show at which particle size of the active ingredient bioequivalence is obtained. The mathematical IVIVC model has been validated internally, i.e. prediction errors (%PE) were calculated for *in vivo* values of the products whose data were used to develop the equation. A similar internal validation was carried out on the prediction of *in vitro* tablet release values based on their correlation with API particle size distribution.

The correlation model is regarded as acceptable when the determination coefficient of the equation $R^2$ is above 0.95 and when the prediction error %PE is below 10%, according to the following formula:

$$\%PE = \frac{\text{observed } in\ vivo \text{ value } - \text{predicted } in\ vivo \text{ value}}{\text{observed } in\ vivo \text{ value}} \times 100\%$$

The obtained IVIVC model equations meet the above criteria, which is confirmed by the data summarized in Table 2. The internal validation of the equation correlating *in vitro* release with API particle size distribution of generic products was also positive, with %PE between -0.65% and 0.97% (data not shown).

Table 2. Predicted *in vivo* values and prediction errors for the series of products used to develop the IVIVC correlation

| **Tablet series** | **Predicted value $c_{max}$ T/R [%] (in brackets PE%) for particular IVIVC models** | | |
|---|---|---|---|
| | **$c_{max}$ T/R = f (F% 2h).** $R^2$ = 0.9915 | **$c_{max}$ T/R = f (F% 3h),** $R^2$ = 0.9993 | **$c_{max}$ T/R = f (F% 4h),** $R^2$ = 0.9905 |
| **Pletal 5564803** | 101.32 (-1.32%) | 100.38 (-0.38%) | 98.56 (1.44%) |
| **CSB021014** | 83.67 (0.44%) | 83.90 (0.16%) | 84.69 (-0.77%) |
| **CSB010715** | 108.33 (0.86%) | 109.01 (0.23%) | 110.05 (-0.71%) |

Based on the obtained equations and the release results of CSB010716 series its bioavailability *in vivo* relative to the reference product was predicted. The results are summarized in Table 3.

Table 3. Predicted in *vivo* value for CSB010716 series based on *in vitro* release results.

| **Tablet series No** | **Predicted value $c_{max}$ T/R [%] for particular IVIVC models** | | |
|---|---|---|---|
| | **$c_{max}$ T/R = f (F% 2h),** $R^2$ = 0.9915 | **$c_{max}$ T/R = f (F% 3h),** $R^2$ = 0.9993 | **$c_{max}$ T/R = f (F% 4h),** $R^2$ = 0.9905 |
| **CSB010716** | 137.67 | 142.40 | 146.96 |

The calculated bioavailability of this series is higher than the bioavailability of the tablets obtained from substances of larger particle size, reported in bioequivalence studies 1 and 2. On the basis of this, unacceptable over-bioavailability of the product can be expected (over the 80.00-125.00% bioequivalence limit).

**Brief Description of Drawings**

The invention will be better explained with support of the drawings, wherein

Fig.1 presents *in vivo* values as the ratio of $c_{max}$ of tested product and reference product $c_{max}$ T/R [%] ± 90% limits of the confidence interval. *In vitro* values are presented as the average amount of API released from the 12 tablets

for each time point ± standard deviation.
Fig. 2 illustrates *in vitro* release results presented as the average value for 12 tablets ± standard deviation, correlated with $D_{0,9}$ particle size of the API used.

## Description of Embodiments

A tablet according to the invention is produced in the following steps:

1. All ingredients are weighed in appropriate amounts and sieved.
2. Ingredients: cilostazol, part of microcrystalline cellulose, part of croscarmellose sodium, starch pregelatinised are mixed and preheated in the fluid bed granulator to the desired temperature 30±10°C. Product temperature is controlled. The hypromellose binder solution is added, prepared by adding hypromellose to the purified water and mixing in appropriate container until dissolved. The mixture is then granulated in the fluid bed granulator to form granules.
3. The granulate is dried in the fluid bed granulator until below 3.0 % moisture is obtained and then, dry granules are calibrated through a screen 2.5 - 3.5 mm size.
4. Sized granules are blended for 10 minutes with the remaining part of microcrystalline cellulose, croscarmellose sodium and magnesium stearate.
5. The final mixture is compressed on a rotary tablet press machine to obtain the desired characteristics of the tablets.
6. Tablets are packaged into blister packs. Filled blisters and leaflets are placed into cartons.

**Example 1**

| Ingredient | Quantity [mg/tablet] |
|---|---|
| Cilostazol | 100.00 |
| Cellulose microcrystalline | 44.50 |
| Starch, pregelatinised | 12.75 |
| Hypromellose | 3.40 |
| Croscarmellose sodium | 8.50 |
| Magnesium stearate | 0.85 |
| Water purified | q. s. |
| **TOTAL** | **170.00** |

Active substance was calibrated initially on the 1.0 mm screen (in order to eliminate aggregates), blended with microcrystalline cellulose type 101, and starch pregelatinised for 3 min in fluid bed granulator. Then fluid bed granulation was conducted with hypromellose solution as granulating agent. After granulation, drying was carried out until below 3.0 % moisture was obtained. After drying the granules were screened through the screen of 1.0 mm size. Later, croscarmellose sodium and MCC type 102 were added. The mixture was blended for 20 min in cubic blender. Then magnesium stearate was added and the whole mixture was blended again for 2 min. The final mixture was compressed on a rotary tablet press machine using round, flat punches, selected for this formulation.

**Example 2**

| Ingredient | Quantity [mg/tablet] |
|---|---|
| Cilostazol | 100.00 |
| Cellulose microcrystalline | 44.50 |
| Starch, pregelatinised | 9.35 |
| Hypromellose | 6.80 |
| Croscarmellose sodium | 8.50 |
| Magnesium stearate | 0.85 |

(continued)

| Ingredient | Quantity [mg/tablet] |
|---|---|
| Water purified | q. s. |
| **TOTAL** | **170.00** |

Active substance was calibrated initially on the 1.0 mm screen (in order to eliminate aggregates), blended with microcrystalline cellulose type 101, and starch pregelatinized for 3 min in fluid bed granulator. Then fluid bed granulation was conducted with hypromellose solution as granulating agent. After granulation, drying was carried out until below 3.0 % moisture was obtained. After drying the granules were screened through the screen of 1.0 mm size. Later, croscarmellose sodium and MCC type 102 were added. The mixture was blended for 20 min in cubic blender. Then magnesium stearate was added and the whole mixture was blended again for 2 min. The final mixture was compressed on a rotary tablet press machine using round, flat punches, selected for this formulation.

**Example 3**

| Ingredient | Quantity [mg/tablet] |
|---|---|
| Cilostazol | 100.00 |
| Cellulose microcrystalline | 44.50 |
| Starch, pregelatinised | 11.05 |
| Hypromellose | 5.10 |
| Croscarmellose sodium | 8.50 |
| Magnesium stearate | 0.85 |
| Water, purified | q. s. |
| **TOTAL** | **170.00** |

Active substance was calibrated initially on the 1.0 mm screen (in order to eliminate aggregates), blended with microcrystalline cellulose type 101, and starch pregelatinized for 3 min in fluid bed granulator. Then fluid bed granulation was conducted with hypromellose solution as granulating agent. After granulation, drying was carried out until below 3.0 % moisture was obtained. After drying the granules were screened through the screen of 1.0 mm size. Later, croscarmellose sodium and MCC type 102 were added. The mixture was blended for 20 min in cubic blender. Then magnesium stearate was added and the whole mixture was blended again for 2 min. The final mixture was compressed on a rotary tablet press machine using round, flat punches, selected for this formulation.

**Example 4**

| Ingredient | Quantity [mg/tablet] |
|---|---|
| Cilostazol | 100.00 |
| Cellulose microcrystalline | 39.40 |
| Starch, pregelatinised | 11.05 |
| Croscarmellose sodium | 13.60 |
| Hypromellose | 5.10 |
| Magnesium stearate | 0.85 |
| Water, purified | q. s. |
| **TOTAL** | **170.00** |

Active substance was calibrated initially on the 1.0 mm screen (in order to eliminate aggregates), blended with microcrystalline cellulose type 101, starch pregelatinized and part of Croscarmellose sodium for 3 min in fluid bed granulator. Then fluid bed granulation was conducted with hypromellose solution as granulating agent. After granulation, drying was carried out until below 3.0 % moisture was obtained. After drying the granules were screened through the

screen of 1.0 mm size. Later, the second part of croscarmellose sodium and MCC type 102 were added. The mixture was blended for 20 min in cubic blender. Then magnesium stearate was added and the whole mixture was blended again for 2 min. The final mixture was compressed on a rotary tablet press machine using round, flat punches, selected for this formulation.

The tablets were used for the development of *in vitro - in vivo* correlation as presented in Table 1.

**Patent Literature**

EP 2481398 A1 (European phase of WO2011037281)
EP2273981 (European phase of WO2009139504)
EP1755682 (WO2005113009)
EP1300160 B1
EP2262487 (WO2009107864)
EP1162973 (WO0057881)
EP1802304 (WO2006030301)

**Non Patent Literature**

- NPL1: Jinno J. et al., Effect of particle size reduction on dissolution and oral absorption of a poorly water-soluble drug, cilostazol, in beagle dogs, J Control Rel. 2006, 111: 56-64.
- Jinno J. et al., In vitro - in vivo correlation for wet-milled tablet of poorly water-soluble cilostazol, J Control Rel. 2008, 130: 29-37.
- Willmann S. et al., Mechanism-based prediction of particle size-dependent dissolution and absorption: Cilostazol pharmacokinetics in dogs, Eur J Pharm Biopharm. 2010, 76: 83-94.
- Miao X. et al., Investigation of Nanosized Crystalline Form to Improve the Oral Bioavailability of Poorly Water Soluble Cilostazol, J Pharm Pharmaceut Sci. 2011, 14: 196-214.

**Claims**

**1.** Pharmaceutical tablet composition comprising a pharmaceutically acceptable ingredient - cilostazol in the form of particles having the following particle size distribution:

$D_{0.5}$ 10-30 μm
$D_{0.9}$ 40-70 μm

and bioavailability of the commercial product reference drug Pletal.

**2.** Pharmaceutical tablet composition of claim 1 comprising cilostazol particles with the following particle size distribution:

$D_{0.5}$ 10-30 μm
$D_{0.9}$ 50-70 μm.

**3.** Pharmaceutical tablet composition according to claim 1 or 2, comprising at least one pharmaceutically acceptable excipient selected from the group of fillers, disintegrants, binders, lubricants.

**4.** Pharmaceutical tablet composition according to claim 3, wherein said filler is selected from: cellulose microcrystalline, powdered cellulose, lactose anhydrous, lactose monohydrate, mannitol, sorbitol, calcium phosphate dibasic anhydrous, calcium phosphate dibasic dehydrate; preferably cellulose microcrystalline.

**5.** Pharmaceutical tablet composition according to claim 3 or 4, wherein said disintegrant is selected from sodium starch glycolate, croscarmellose or its salt, crospovidone, hydroxypropyl cellulose low-substituted, starch, starch pregelatinized; preferably croscarmellose sodium.

6. Pharmaceutical tablet composition according to any claim of 3-5, wherein said binder is selected from povidone, hydroxypropylcellulose, hydroxypropyl methylcellulose (hypromellose); preferably hydroxypropyl methylcellulose.

7. Pharmaceutical tablet composition according to any claim of 3-6, wherein said lubricant is selected from stearic acid, stearic acid salts such as magnesium stearate, sodium lauryl sulfate, hydrogenated vegetable oil, hydrogenated castor oil; preferably magnesium stearate.

8. Pharmaceutical composition according to any claim of 3-7, which is obtained by tableting the granulate manufactured in wet- granulation process.

Fig. 1

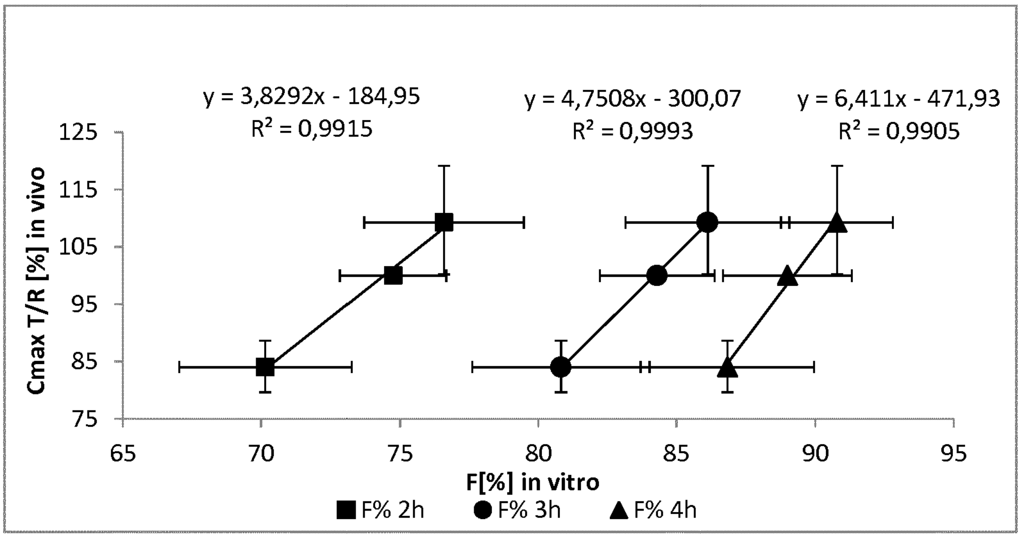
y = 3,8292x - 184,95
R² = 0,9915
y = 4,7508x - 300,07
R² = 0,9993
y = 6,411x - 471,93
R² = 0,9905
Cmax T/R [%] in vivo
125
115
105
95
85
75
65
70
75
80
85
90
95
F[%] in vitro
F% 2h
F% 3h
F% 4h

Fig. 2

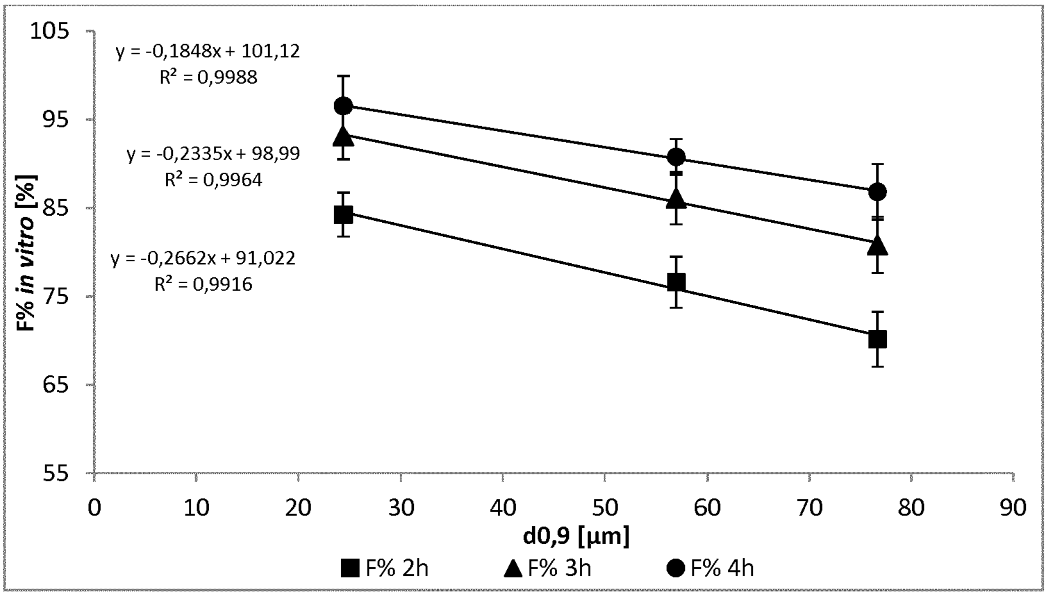
y = -0,1848x + 101,12
R² = 0,9988
y = -0,2335x + 98,99
R² = 0,9964
y = -0,2662x + 91,022
R² = 0,9916
F% in vitro [%]
105
95
85
75
65
55
0
10
20
30
40
50
60
70
80
90
d0,9 [µm]
F% 2h
F% 3h
F% 4h

Europäisches Patentamt
European Patent Office
Office européen des brevets

# EUROPEAN SEARCH REPORT

Application Number
EP 17 46 1543

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/030301 A1 (RANBAXY LAB LTD [IN]; MURPANI DEEPAK [IN]; BHATTI ASHIMA [IN]) 23 March 2006 (2006-03-23)<br>* page 1 - page 2; claims 3,11,16,17,19,20; examples 1,2 *<br>----- | 1-8 | INV.<br>A61K47/38<br>A61K9/20<br>A61K9/28<br>A61K31/4709<br>A61K9/14 |
| A | WILLMANN S ET AL: "Mechanism-based prediction of particle size-dependent dissolution and absorption: Cilostazol pharmacokinetics in dogs",<br>EUROPEAN JOURNAL OF PHARMACEUTICS AND BIOPHARMACEUTICS, ELSEVIER SCIENCE PUBLISHERS B.V., AMSTERDAM, NL,<br>vol. 76, no. 1,<br>1 September 2010 (2010-09-01), pages 83-94, XP027210086,<br>ISSN: 0939-6411<br>[retrieved on 2010-06-08]<br>* the whole document *<br>----- | 1-8 | |
| A | JINNO J I ET AL: "Effect of particle size reduction on dissolution and oral absorption of a poorly water-soluble drug, cilostazol, in beagle dogs",<br>JOURNAL OF CONTROLLED RELEASE, ELSEVIER, AMSTERDAM, NL,<br>vol. Effect of particle size reduction on dissoluti111, no. 1-2,<br>10 March 2006 (2006-03-10), pages 56-64, XP024957430,<br>ISSN: 0168-3659, DOI: 10.1016/J.JCONREL.2005.11.013<br>[retrieved on 2006-03-10]<br>* the whole document *<br>----- | 1-8 | TECHNICAL FIELDS SEARCHED (IPC)<br>A61K |

The present search report has been drawn up for all claims

1

| Place of search | Date of completion of the search | Examiner |
|---|---|---|
| Munich | 16 August 2017 | Kardas-Llorens, Eyüp |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 46 1543

This annex lists the patent family members relating to the patent documents cited in the above-mentioned European search report. The members are as contained in the European Patent Office EDP file on
The European Patent Office is in no way liable for these particulars which are merely given for the purpose of information.

16-08-2017

| Patent document cited in search report | Publication date | Patent family member(s) | Publication date |
|---|---|---|---|
| WO 2006030301 A1 | 23-03-2006 | EP 1802304 A1 | 04-07-2007 |
| | | US 2008206348 A1 | 28-08-2008 |
| | | WO 2006030301 A1 | 23-03-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2481398 A1
- WO 2011037281 A
- EP 2273981 A
- WO 2009139504 A
- EP 1755682 A
- WO 2005113009 A
- EP 1300160 B1
- EP 2262487 A
- WO 2009107864 A
- EP 1162973 A
- WO 0057881 A
- EP 1802304 A
- WO 2006030301 A

### Non-patent literature cited in the description

- **JINNO J. et al.** Effect of particle size reduction on dissolution and oral absorption of a poorly water-soluble drug, cilostazol, in beagle dogs. *J Control Rel.,* 2006, vol. 111, 56-64
- **JINNO J. et al.** In vitro - in vivo correlation for wet-milled tablet of poorly water-soluble cilostazol. *J Control Rel.,* 2008, vol. 130, 29-37
- **WILLMANN S. et al.** Mechanism-based prediction of particle size-dependent dissolution and absorption: Cilostazol pharmacokinetics in dogs. *Eur J Pharm Biopharm.,* 2010, vol. 76, 83-94
- **MIAO X. et al.** Investigation of Nanosized Crystalline Form to Improve the Oral Bioavailability of Poorly Water Soluble Cilostazol. *J Pharm Pharmaceut Sci.,* 2011, vol. 14, 196-214